# EUROPEAN PATENT APPLICATION

(11) **EP 1 930 015 A1**
(43) Date of publication of application: **11.06.2008**
(21) Application number: 06450176.0
(22) Date of filing: 05.12.2006
(51) Int. Cl.: A61K 31/731, A61P 31/16, A61K 31/737

(54) **Use of carrageenan for treating rhinovirus infections**

(71) Applicant: Marinomed Biotechnologie GmbH, 1210 Vienna (AT)
(72) Inventor: Grassauer, Andreas, 1220 Vienna (AT); Meier, Christiane, 2201 Seyring (AT); Prieschl-Grassauer, Eva, 1220 Vienna (AT); Pretsch, Alexander, 2002 Roseldorf (AT)
(74) Representative: Sonn & Partner Patentanwälte

(57) **Abstract**

The present invention provides the use of carrageenan or mixtures thereof for the manufacture of an antiviral pharmaceutical composition for the treatment of rhinovirus infections.

## Description

The present invention relates to the field of immunology and antiviral agents.

Picornaviruses represent a very large virus family of small ribonucleic acid-containing viruses responsible for many serious human and animal diseases. Picornaviruses include four major groups: enteroviruses, rhinoviruses, cardioviruses and apthoviruses.

The human rhinoviruses consist of at least 100 serotypes and are the primary causative agents of the common cold. Because of the large number of serotypes, development of a vaccine is problematic; antiviral agents may therefore be the best approach to treatment. Rhinoviruses are composed of a surrounding capsid,which contains four viral proteins VP1, VP2, VP3 and VP4. Proteins VP1, VP2 and VP3 are organised into 60 repeating protameric icosahedral units. These are thought to be the cause of antigen diversity associated with these viruses.

Rhinovirus (HRV) infections lead to the common cold with symptoms such as fever, cough, and nasal congestion. HRV infection is the second most frequently recognised agent associated with pneumonia and bronchiolitis in infants and young children and commonly causes exacerbations of pre-existing airways disease in those with asthma, chronic obstructive pulmonary disease or cystic fibrosis. HRV infection is associated with one-third to one-half of asthma exacerbations depending on age and is linked to asthma hospitalisations in both adults and children.

Present treatment approaches include the application of rhinovirus specific RNA, as disclosed in the DE 19825395, which binds to the canyon region of the capsid, which is necessary to host receptor (e.g. ICAM-1, intercelluar adhesion molecule-1) binding and cell infection.

Another method comprises the administration of soluble ICAM-1 proteins or derivatives of ICAM-1 as disclosed in the US6,326,004 and the US 6,096,862 (tICAM-1, truncated ICAM-1) to neutralize viral particles (virions).

As disclosed in US 2003/181415 A sulfated polysaccharides, including cellulose sulfate, are known to be effective against various enveloped viruses and in particular herpes simplex virus (HSV), Papilloma viruses and HIV.

Chemical compounds with antiviral activity against rhinoviruses are disclosed in the EP 0523803.

Nevertheless, still the dominant treatment forms aim at the symptoms than the origins of the infection. Rhinovirus symptoms are caused by an over or unspecific reaction of the immune system. Therefore common treatment forms of a rhinovirus infection include administration of analgesics such as aspirin or acetaminophen/ paracetamol, as well as localised versions targeting the throat (often delivered in lozenge form), nasal decongestants which reduce the inflammation in the nasal passages by constricting local blood vessels, cough suppressants (which work to suppress the cough reflex of the brain or by diluting the mucus in the lungs), and first-generation anti-histamines such as brompheniramine, chlorpheniramine, and clemastine (which reduce mucus gland secretion and thus combat blocked/runny noses but also may make the user drowsy).

It is a goal of the present invention to provide a pharmaceutical composition for effective treatment of rhinitis.

The present invention provides the use of a sulfated polymer for the manufacture of an antiviral pharmaceutical composition for the treatment of rhinovirus infections (rhinitis) or prophylaxis of rhinovirus infections, wherein the polymer is selected from carrageen, preferably lambda- iota or kappa-carrageenan, or mixtures thereof. There exist more than 10 different carrageenan forms, where from lambda- iota or kappa-carrageenan, especially iota-carrageenan were the most effective. The preparation is especially suitable for the treatment of a rhinovirus infection, especially for acute or chronic rhinovirus infections. Carrageenan is a generic term for several polysaccharides extracted from seaweed (Rhodophyceae). Carrageenan compounds differ from agar in that they have sulfated groups (-OSO₃) in place of some hydroxyl groups; with 1beta-3-, 1alpha- 4-,3,6-anhydro-D-galatose. Synonyms of carrageenan are irish moss gelose (from Chondrus spp.); Eucheuman (from Eucheuma spp.); Iridophycan (from Iridae spp.); Hypnean (from Hypnea spp.); Furcellaran or Danish agar (from Furcellaria fastigiata); INS No. 407. The preferred carrageenan forms are: kappa-carrageenan which forms strong, rigid gels; predominantly produced from Kappaphycus cottonii; iota-carrageenan which forms soft gels and is predominantly produced from Eucheuma spinosum and lambda-carrageenan, which is the most common form used to thicken dairy products. Carrageenan is used as thickener, gelling agent, stabilizer and emulsifier in pharmaceutical and food products.

It was now surprisingly found that carrageenan had antiviral activity, with iota-carrageenan showing the best activity, especially against rhinovirus infections (rhinitis) - as active principle for therapy or prophylaxis. Prophylaxis as used herein relates to the reduction of the risk of an infection. Also disclosed is the use of carrageenan for the manufacture of a pharmaceutical composition or a composition for a medicinal product for the treatment or prevention of the symptoms of a rhinovirus infection.

In a preferred embodiment of the present invention the selected carrageenan form is purified, in particular sterilely purified, e.g. through filtration through a sterilisation filter in soluble form.

Preferably the pharmaceutical preparation is in form of a preparation for topical or mucosal use, preferably skin lotions, cremes, powders, sprays or gargle solutions. The carrageenan preparation may be fluid or solid, e.g. a dry powder. Preparations for mucosal application include nose sprays or drops, e.g. in form of a fluid solution, or other nasal drug delivery systems known in the state of the art, e.g. from the US 6,391,452. The polymer is especially suitable for topical application to treat skin or mucosal inflammation. But also systemic, e.g. parenteral or oral (also for specific mucosal treatment), is possible, especially for low molecular weight polymers. The present invention also provides the use of the pharmaceutical preparations.

In other embodiments the present invention provides hygiene or sanitation articles comprising the polymer (carrageenan selected from lambda-, iota- or kappa-carrageenan or mixtures thereof), as homo- or heteropolymers, preferably tissues or handkerchiefs. In particular the hygiene or sanitation article is a facial hygiene or sanitation article, preferably for the oral and/or nasal area, especially preferred being for topical or mucosal use, most preferred being selected from nose sprays, powders, drops or gargle solutions. The polymer can be easily incorporated into the tissue material for antiviral effects. Further articles with the polymer are e.g. cotton swabs, lipsticks, dust masks or facial masks. These hygiene or sanitation articles can be used prophylactically or as treatment form against a rhinovirus infection. The use of the articles can result in the prevention or reduction of the risk of the infection.

Preferably, the polymer has a molecular weight ranging from about 15,000 to 5,000,000 Da, preferably greater than 50,000 Da, in particular between 50,000 and 3,000,000 Da.

Also preferred is that the preparation comprises pharmaceutical carriers or additives. The term "carrier" refers to a diluent, e.g. water, saline, excipient, or vehicle with which the composition can be administered. For a solid or fluid composition the carriers or additives in the pharmaceutical composition may comprise SiO₂, TiO₂, a binder, such as microcrystalline cellulose, polyvinylpyrrolidone (polyvidone or povidone), gum tragacanth, gelatine, starch, lactose or lactose monohydrate, alginic acid, maize starch and the like; a lubricant or surfactant, such as magnesium stearate, or sodium lauryl sulphate; a glidant, such as colloidal silicon dioxide; a sweetening agent, such as sucrose or saccharin. Preferably the preparation comprises buffers or pH adjusting agents, e.g. selected from citric acid, acetic acid, fumaric acid, hydrochloric acid, malic acid, nitric acid, phosphoric acid, propionic acid, sulfuric acid, tartaric acid, or combinations thereof. Carrageenan in the form of a pharmaceutically acceptable salt, for example sodium salts may be used. Other pharmaceutically acceptable salts include, among others, potassium, lithium and ammonium carrageenan.

Furthermore, preferably the iota, lambda- or kappa-carrageenan is in amounts between 0.01% and 20%, preferably between 0.1% and 10%, most preferred between 0.5% and 5% of the preparation (%-values are given in w/w-%). In the pharmaceutical preparation also other anti-viral polymers than carrageenan can be present, most preferably cellulose sulfate. For these polymers the same preferred concentration ranges apply.

The polymer is preferably a carrageenan homopolymer, e.g. molecular pure carrageenan of one form (preferably iota, kappa or lambda), or alternatively a derivative heteropolymer. Heteropolymers are for example of different carrageenan subunits, especially a heteropolymer of iota, kappa- or lambda-carrageenan subunits.

In specific embodiments the preparation is substantially free of other carrageenan forms than the preferred- although trace amount can be present. Preferably, in the preparation iota-carrageenan is substantially the only form of carrageenan. The antiviral effective carrageenan forms (kappa, lambda, and/or iota) are e.g. more than 80%, preferably more than 90%, especially preferred more than 95%, most preferred more than 99% (percentages given in w/w-%) of the carrageenan forms (individually or in combination), or in another embodiment of the anti-viral active principles. In other embodiments iota-carrageenan composes more than 50%, preferably more than 70%, especially preferred more than 80%, most preferred more than 95% of the carrageenan forms - in a heteropolymer or a homopolymer. The given values can apply either to a heteropolymer or a homopolymer.

The administration of the preparation is not limited to administrations at the same time of a disease but can also be used before or after an infection, e.g. for prophylactic treatment, i.e. a treatment before an expected infection to reduce the force of the disease. In the preparation only one anti-viral agent, e.g. iota-carrageenan, may be present. Nevertheless, in a more preferred embodiment the pharmaceutical preparation comprises at least two or three different anti-viral components, preferably one component is cellulose sulfate. Cellulose sulfate is an exceptional anti-viral polymer (alone or in combination), which can be used in a preparation of the present invention.

In further embodiments carrageenan is used as additive in a medical formulation. Carrageenan as active principle against rhinovirus infections can be used in divers medicinal products as additive such as carrier, emulsifier or viscosity modifying agent since it is compatible with most pharmaceutical preparations and without side-effects. Therefore, as additional use almost any medicament can be enhanced with an anti-rhinovirus function by the use of a carrageenan as additive.

In another embodiment the pharmaceutical preparation is for the treatment or prophylaxis in asthma patients. Asthma patienties are especially susceptible to or are at risk for a rhinovirus infection and are thus preferred patients for a treatment with a carrageenan preparation.

The present invention is further illustrated by the following figures and examples.

Figures:

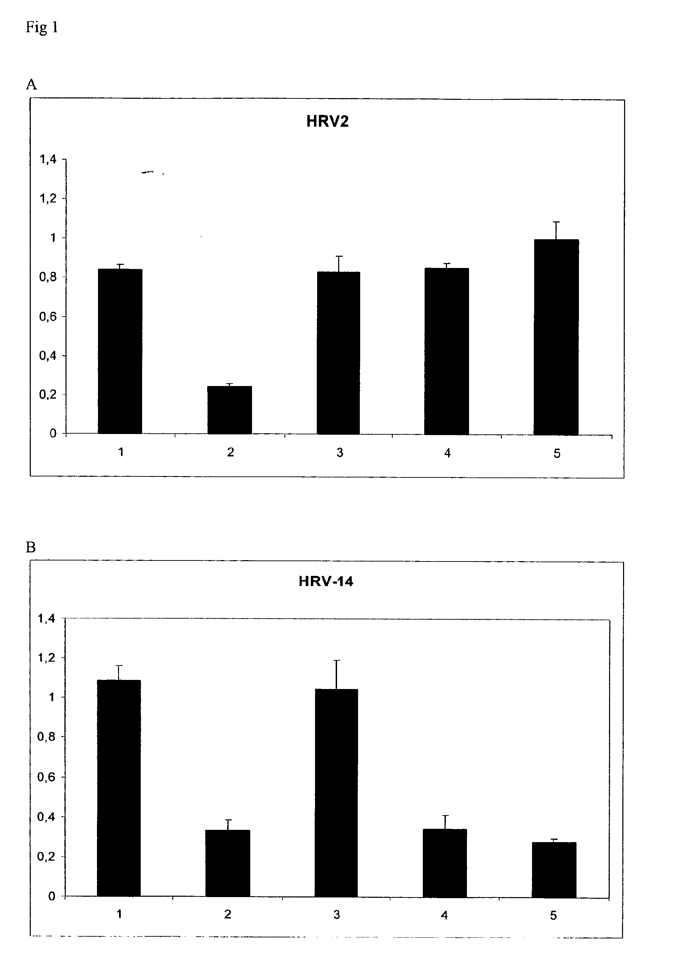

Fig. 1: HRV induced cell death inhibition assay (XTT-assay) y-axis: OD 492nm, 1 uninfected cells 2 untreated cells, 3 iota-Carrageenan , 4 kappa-Carrageenan, 5 lambda- Carrageenan. Fig.1A HRV-2, Fig.1B HRV-14

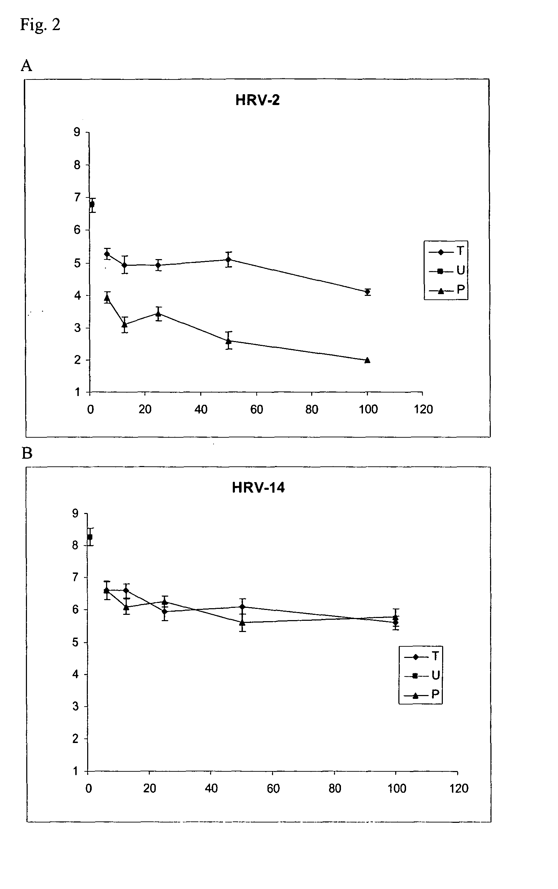

Fig. 2: Peak titers of supernatants from infected Hela cells were determined by TCID₅₀ assays. The Y-Axis indicates the TCID₅₀ titer. The x-Axis indicates the concentration of iota-Carageenan in µg/ml. T (treatment) indicates that cells were infected for one hour and the indicated concentration of iota-carageenan was added one hour after treatment. P (prophylaxis) indicates that the virus suspension was preincubated with the indicated concentration of iota-carageenan for on hour before infection. Further processing was identical to T. Cells were infected at a multiplicity of infection of 0,01. The peak titers were observed at day 3 for HRV-2 (Fig. 2A) and at day 4 for HRV-14 (Fig. 2B).

### Examples:

### Example 1: Effect of different types of carageenans against human rhinovirus type 2 and type 14:

Subconfluent HeLa cells were incubated with a virus suspension that was preincubated for 5 min with 125 µg/ml of the indicated polymers. 48 hours later the viability of the cells was determined with TOX2 XTT assay (Sigma). (Fig. 1). Error bars indicate the standard deviation between six independent wells. The most effective polymer was iota-carrageenan that was effective against both types of rhinovirus while, lambda-carrageenan and kappa-carrageenan showed effectivity for HRV-2 but not for HRV-14.

### Example 2:

The polymers indicated in Table 1 were tested in a HRV-2 and HRV-14 induced cell death inhibition assay (XTT-assay) at a concentration of 100µg/ml: iota Carageenan showed protection against both types of Rhinovirus. Kappa Carageenan and lambda carrageenan, were active against HRV-2 but not against HRV-14. The polymers Chitosan, Carboxymethylcellulose and Carboxymethylchitosan did not show an inhibitory effect.

### Example 3:

Iota-Carageenan is effective against HRV-2 and HRV-14 in a treatment and a prophylaxis viral replication model. A significant reduction of peak viral titer was observed at concentrations equal and higher than 6,25µg/ml. However, iota carageenan was most effective in the prophylaxis model against HRV-2 in which a reduction of more than 99,9% in the peak viral titer was observed.

### Example 4:

Several polymers were tested for their activity against HRV-2 and HRV-14 in HRV induced cell death inhibition assays at a concentration of 100µg/ml. + indicates at least 95% protection when compared to unifected control cells.

**Table 1: Antiviral activity of several tested polymers**

| Polymer | HRV-2 | HRV-14 |
|---|---|---|
| Iota-Carrageenan | + | + |
| Kappa-Carrageenan | + | - |
| Lambda-Carrageenan | + | - |
| Chitosan | - | - |
| Carboxymethylcellulose | - | - |
| Carboxymethylchitosan | - | - |

## Claims

1. Use of carrageenan for the manufacture of an antiviral pharmaceutical composition for the treatment or prophylaxis of rhinovirus infections or prophylaxis of rhinovirus infections.

2. Use according to claim 1, **characterized in that** the preparation is in form of a preparation for topical or mucosal use, preferably nose sprays, powders, drops or gargle solutions.

3. Use according to claim 1 or 2, **characterized in that** the preparation comprises pharmaceutical carriers or additives.

4. Use according to any one of claims 1 to 3, **characterized in that** carrageenan is in amounts between 0,01% and 20%, preferably between 0,1% and 10%, most preferred between 0,5% and 5% of the preparation.

5. Use according to any one of claims 1 to 4, **characterized in that** the preparation comprises at least two different antiviral' components, preferably one component is cellulose sulfate.

6. Use according to any one of claims 1 to 5, **characterized in that** the preparation is sterile.

7. Use according to any one of claims 1 to 6, **characterized in that** the in the preparation lambda-,kappa- and iota-carrageenan comprise more than 80%, preferably more than 90%, more preferred more than 95%, most preferred more than 99% of all carrageenan forms of the preparation.

8. Use according to any one of claims 1 to 7, **characterized in that** in the preparation iota-carrageenan comprises more than 80%, preferably more than 90%, more preferred more than 95%, most preferred more than 99% of all carrageenan forms of the preparation.

9. Use according to any one of claims 1 to 6, **characterized in that** carrageenan is a lambda-carrageenan homopolymer.

10. Use according to any one of claims 1 to 6, **characterized in that** carrageenan is a kappa-carrageenan homopolymer.

11. Use according to any one of claims 1 to 6, **characterized in that** carrageenan is a iota-carrageenan homopolymer.

12. Use according to any one of claims 1 to 11, **characterized in that** the preparation is for the treatment of a rhinovirus infection, preferably an acute or a chronic rhinovirus infection.

13. Use according to any one of claims 1 to 12, **characterized in that** carrageenan is used as additive in a medical formulation.

14. Use according to any one of claims 1 to 13, **characterized in that** the medicament is for the treatment or prophylaxis in asthma patients.

15. Hygiene or sanitation article comprising carrageenan selected from iota-, lambda- or kappa-carrageenan or mixtures thereof, preferably as homopolymers.

16. Hygiene or sanitation article according to claim 15, being a facial hygiene or sanitation article, preferably for the oral and/or nasal area, especially preferred being for topical or mucosal use, most preferred being selected from nose sprays, powders, drops or gargle solutions.
